(19) Europäisches Patentamt
European Patent Office
Office européen des brevets

(11) **EP 3 647 771 B1**

(12) # EUROPEAN PATENT SPECIFICATION

(45) Date of publication and mention
of the grant of the patent:
**08.04.2026 Bulletin 2026/15**

(21) Application number: **17915268.1**

(22) Date of filing: **29.06.2017**

(51) International Patent Classification (IPC):
**G06V 20/10** (2022.01) **G06V 10/764** (2022.01)
**G06F 18/2453** (2023.01) **G01N 21/359** (2014.01)
**A01G 7/00** (2006.01)

(52) Cooperative Patent Classification (CPC):
**G06V 20/188; A01G 7/00; G01N 21/359;
G06F 18/2453; G06V 10/764**

(86) International application number:
**PCT/JP2017/024038**

(87) International publication number:
**WO 2019/003400 (03.01.2019 Gazette 2019/01)**

(54) **COEFFICIENT CALCULATION DEVICE, COEFFICIENT CALCULATION METHOD, AND RECORDING MEDIUM IN WHICH COEFFICIENT CALCULATION PROGRAM IS RECORDED**

KOEFFIZIENTBERECHNUNGSVORRICHTUNG, KOEFFIZIENTBERECHNUNGSVERFAHREN UND AUFZEICHNUNGSMEDIUM MIT DARAUF AUFGEZEICHNETEM KOEFFIZIENTBERECHNUNGSPROGRAMM

DISPOSITIF DE CALCUL DE COEFFICIENT, PROCÉDÉ DE CALCUL DE COEFFICIENT ET SUPPORT D'ENREGISTREMENT DANS LEQUEL EST ENREGISTRÉ UN PROGRAMME DE CALCUL DE COEFFICIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**06.05.2020 Bulletin 2020/19**

(73) Proprietor: **NEC Corporation
Minato-ku
Tokyo 108-8001 (JP)**

(72) Inventors:
• **FUJIYAMA Kenichiro**
**Tokyo 108-8001 (JP)**
• **SATOH Mineto**
**Tokyo 108-8001 (JP)**
• **AZUMA Tan**
**Tokyo 108-8001 (JP)**
• **ARIYAMA Tetsuri**
**Tokyo 108-8001 (JP)**

(74) Representative: **Betten & Resch
Patent- und Rechtsanwälte PartGmbB
Maximiliansplatz 14
80333 München (DE)**

(56) References cited:
**WO-A1-2014/103181      WO-A1-2016/203689
JP-A- 2006 085 517      JP-A- 2010 117 327
JP-A- 2010 220 569      JP-A- 2013 138 811
JP-A- 2014 183 788      JP-A- 2016 142 633
US-A1- 2007 065 857      US-A1- 2009 007 485
US-A1- 2013 152 464**

• **G.S. CAMPBELL: "Extinction coefficients for radiation in plant canopies calculated using an ellipsoidal inclination angle distribution", AGRICULTURAL AND FOREST METEOROLOGY., vol. 36, no. 4, 1 April 1986 (1986-04-01), NL, pages 317 - 321, XP055701267, ISSN: 0168-1923, DOI: 10.1016/0168-1923(86) 90010-9**

**(Cont. next page)**

- WANG ET AL: "Comparison of leaf angle distribution functions: Effects on extinction coefficient and fraction of sunlit foliage", AGRICULTURAL AND FOREST METEOROLOGY, ELSEVIER, AMSTERDAM, NL, vol. 143, no. 1-2, 9 February 2007 (2007-02-09), pages 106 - 122, XP005880509, ISSN: 0168-1923, DOI: 10.1016/ J.AGRFORMET.2006.12.003
- TAKU M. SAITOH ET AL: "Examination of the extinction coefficient in the Beer-Lambert law for an accurate estimation of the forest canopy leaf area index", FOREST SCIENCE AND TECHNOLOGY, vol. 8, no. 2, 1 June 2012 (2012-06-01), pages 67 - 76, XP055699470, ISSN: 2158-0103, DOI: 10.1080/21580103.2012.673744
- D. PARAMELLE ET AL: "A rapid method to estimate the concentration of citrate capped silver nanoparticles from UV-visible light spectra", ANALYST, vol. 139, no. 19, 1 July 2014 (2014-07-01), UK, pages 4855 - 4861, XP055701277, ISSN: 0003-2654, DOI: 10.1039/ C4AN00978A
- TAKUYA SHIMOJO: "Light penetration rate visualization in tomato foliage using wireless sensor networks", IEICE TECHNICAL REPORT, vol. 112, no. 464 (IN2012-215), 2013, pages 365 - 370, XP009517919, ISSN: 0913-5685
- CHUFENG HU ET AL: "The polarized differential extinction coefficient with frequency determined by morphological characteristics of forest vegetation", 2016 IEEE INTERNATIONAL GEOSCIENCE AND REMOTE SENSING SYMPOSIUM (IGARSS), 10 July 2016 (2016-07-10) - 15 July 2016 (2016-07-15), pages 1734 - 1737, XP032990663, DOI: 10.1109/ IGARSS.2016.7729444

**Description**

[Technical Field]

The present invention relates to a coefficient calculation device and the like that calculates an extinction coefficient for a certain area.

[Background Art]

Quality of farming implemented in a field can be measured, for example, by using a difference between an event predicted to be caused by implementing farming in the field and an event caused by actually implementing farming in the field. In a case of measuring the event by a leaf area, first, an irrigation amount or the like is predicted based on a content of the farming implemented in the field, and a leaf area of a plant growing in the field is predicted based on the irrigation amount. Next, the leaf area of the plant vegetating in the field is actually measured, and the quality of the farming implemented in the field can be measured in response to whether or not the measured leaf area is equal to or more than the predicted area. In a case of measuring the leaf area or the like of the plant actually vegetating in the field, for example, a radar image taken at an angle of view of the field is sometimes used as illustrated in PTL 1.

PTL 1 discloses an analysis device that calculates a degree of vegetation in a target area, based on a radar image in which a state of a ground surface of the target area is taken. The analysis device calculates a backscattering coefficient for a specific area in a target area, based on a radar image in which the target area is taken during a predetermined time period and a radar image in which the target area is taken during another time period, and calculates, based on a correlation between the backscattering coefficient and the degree of the vegetation, a degree of vegetation in the specific area.

NPL 1 models plant canopy extinction coefficients using an ellipsoidal leaf angle distribution. Derives equations for K versus beam elevation and parameter x, provides a polynomial approximation, and validates against measured leaf angle classes. Offers a simple yet accurate method to simulate radiation attenuation in diverse canopy architectures.

[Citation List]

[Patent Literature]

PTL 1: Japanese Patent Application Publication No. 2010-117327
NPL 1: G.S. CAMPBELL: "Extinction coefficients for radiation in plant canopies calculated using an ellipsoidal inclination angle distribution", AGRICULTURAL AND FOREST METEOROLOGY., vol. 36, no. 4, 1 April 1986 (1986-04-01), pages 317-321

[Summary of Invention]

[Technical Problem]

A leaf area can be predicted, for example, based on a vegetation index (WDVI) of a plant vegetating in a field and an extinction coefficient in the field. In this case, the leaf area is predicted more correctly as the extinction coefficient is more accurate. The extinction coefficient is a value that cannot be measured, and accordingly, is calculated based on other pieces of information. However, a large calculation amount is required for calculating an accurate extinction coefficient.

In view of the above, an object of the present invention is to provide a coefficient calculation device or the like capable of calculating an accurate extinction coefficient for a certain area in a short period.

[Solution to Problem]

The solution is presented in the appended claims.

As an aspect of the present invention, a coefficient calculation device includes:

first coefficient calculation means for calculating an extinction coefficient for a part of partial areas among a plurality of partial areas in a field in accordance with a predetermined calculation processing;
relevance information generation means for generating relevance information representing a relevance between the extinction coefficient calculated for the part of partial areas and a characteristic value representing a character of the part of partial areas; and

second coefficient calculation means for calculating an extinction coefficient for another partial area different from the part of partial areas among the plurality of partial areas based on the characteristic value for the another partial area and the relevance information generated by the relevance information generation means.

In addition, as another aspect of the present invention, a coefficient calculation method includes:

calculating an extinction coefficient for a part of partial areas among a plurality of partial areas in a field in accordance with a predetermined calculation processing;

generating relevance information representing a relevance between the extinction coefficient calculated for the part of partial areas and a characteristic value representing a character of the part of partial areas; and

calculating an extinction coefficient for another partial area different from the part of partial areas among the plurality of partial areas based on the characteristic value for the another partial area and the generated relevance information.

In addition, as another aspect of the present invention, a coefficient calculation program causing a compute to achieve:

a first coefficient calculation function for calculating an extinction coefficient for a part of partial areas among a plurality of partial areas in a field in accordance with a predetermined calculation processing;

a relevance information generation function for generating relevance information representing a relevance between the extinction coefficient calculated for the part of partial areas and a characteristic value representing a character of the part of partial areas; and

a second coefficient calculation function for calculating an extinction coefficient for another partial area different from the part of partial areas among the plurality of partial areas based on the characteristic value for the another partial area and the relevance information generated by the relevance information generation function.

Furthermore, the object is also achieved by a computer-readable recording medium that records the program.

[Advantageous effects of Invention]

In accordance with the coefficient calculation device according to the present invention, an accurate extinction coefficient for a certain area can be calculated in a short period.

[Brief Description of Drawings]

[Fig. 1] Fig. 1 is a block diagram illustrating a configuration of a coefficient calculation device according to a first example embodiment of the present invention.

[Fig. 2] Fig. 2 is a flowchart illustrating a flow of processing of the coefficient calculation device according to the first example embodiment.

[Fig. 3] Fig. 3 is a view conceptually illustrating examples of image information and partial image information.

[Fig. 4] Fig. 4 is a diagram conceptually illustrating an example of information generated by a relevance information generation unit.

[Fig. 5] Fig. 5 is a diagram conceptually illustrating an example of calculated relevance information.

[Fig. 6] Fig. 6 is a block diagram illustrating a configuration of a coefficient calculation device according to a second example embodiment of the present invention.

[Fig. 7] Fig. 7 is a flowchart illustrating a flow of processing of the coefficient calculation device according to the second example embodiment.

[Fig. 8] Fig. 8 is a block diagram illustrating a configuration of a coefficient calculation device according to a third example embodiment of the present invention.

[Fig. 9] Fig. 9 is a flowchart illustrating a flow of processing of the coefficient calculation device according to the third example embodiment.

[Fig. 10] Fig. 10 is a diagram conceptually illustrating an example of relevance information calculated for each group.

[Fig. 11] Fig. 11 is a block diagram illustrating a configuration of a coefficient calculation device according to a fourth example embodiment of the present invention.

[Fig. 12] Fig. 12 is a flowchart illustrating a flow of processing of the coefficient calculation device according to the fourth example embodiment.

[Fig. 13] Fig. 13 is a block diagram schematically illustrating a hardware configuration of a calculation processing device capable of achieving a coefficient calculation device according to each example embodiment of the present invention.

[Example Embodiment]

Next, example embodiments of the present invention will be described in detail with reference to drawings.

<First example embodiment>

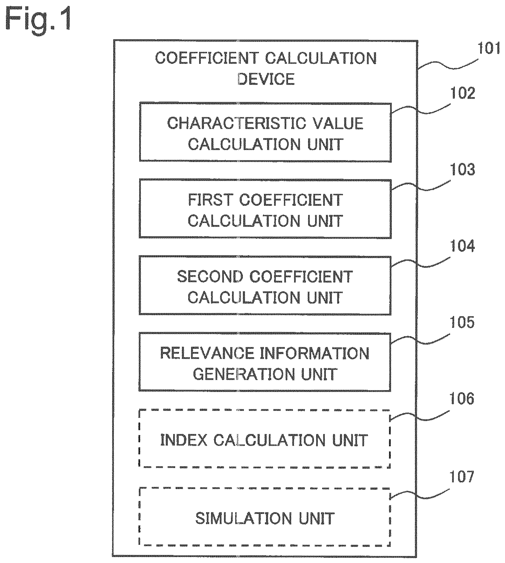

Referring to Fig. 1, a configuration of a coefficient calculation device 101 according to a first example embodiment of the present invention will be described in detail. Fig. 1 is a block diagram illustrating the configuration of the coefficient calculation device 101 according to the first example embodiment of the present invention.

The coefficient calculation device 101 according to the first example embodiment includes a characteristic value calculation unit (characteristic value calculator) 102, a first coefficient calculation unit (first coefficient calculator) 103, a second coefficient calculation unit (second coefficient calculator) 104, and a relevance information generation unit (relevance information generator) 105. The coefficient calculation device 101 may further include an index calculation unit (index calculator) 106 and a simulation unit (simulator) 107.

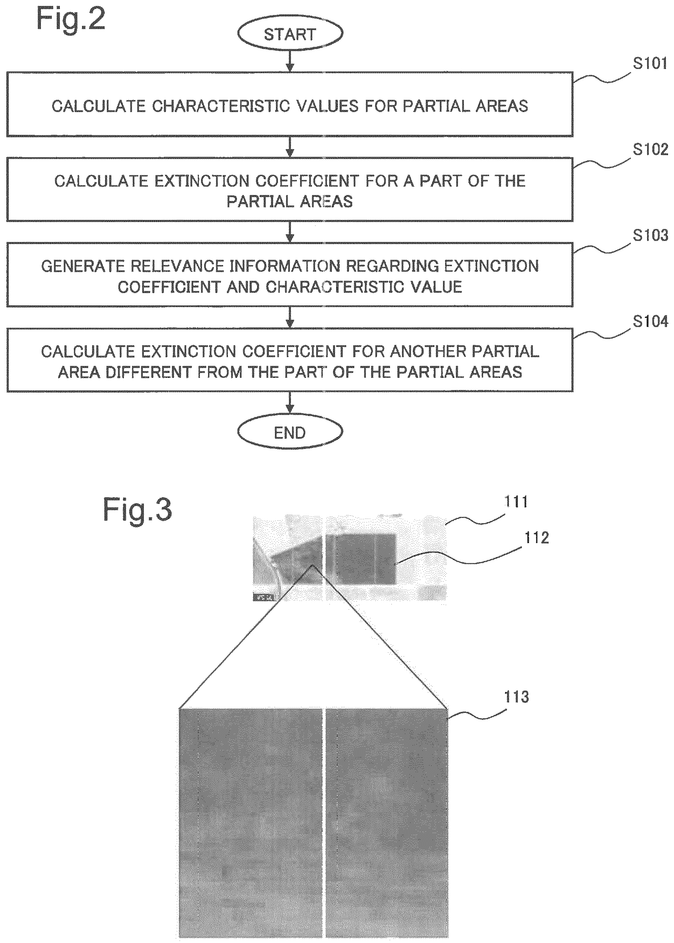

The coefficient calculation device 101 receives partial image information 113 (Fig. 3) from a sensor, a storage device, or the like. The partial image information 113 represents an image, which is obtained by dividing a field area into a plurality of partial areas, in image information 111 (Fig. 3) taken at an angle of view of a field 112 (Fig. 3). Fig. 3 is a view conceptually illustrating examples of the image information 111 and the partial image information 113.

Processing to be described later with reference to Fig. 2 is executed for the partial area represented by the partial image information 113.

The image information 111 is, for example, an image taken at an angle of view of the field 112 by using a near infrared camera mounted on an artificial satellite, a drone, or a helicopter. The image information 111 may include information representing an area other than the field 112. Moreover, the image information 111 may be a plurality of images taken for one field, such as an image in which a visible region is taken and an image in which a near infrared region is taken. For convenience of explanation, information representing an area of the field 112 (hereinafter, referred to as "field area") in the image information 111 will be hereinafter referred to as "field area information".

The plurality of partial areas may or may not include an area of overlapping each other. Moreover, the partial areas may have a regular shape or may have an irregular shape. For convenience of explanation, in the description below, it is assumed that the partial areas are areas arranged in a grid pattern in the field area, and that the plurality of partial areas do not include the area of overlapping each other.

Further, the coefficient calculation device 101 may capture the image information 111 taken at an angle of view of the field 112, and may specify the field area information representing the field 112 from the image information 111. In this case, the coefficient calculation device 101 divides the field area, which is represented by the specified field area information, into the plurality of partial areas and, thereby, generates the partial image information 113 representing each partial area.

Next, referring to Fig. 2, processing of the coefficient calculation device 101 according to the first example embodiment of the present invention will be described in detail. Fig. 2 is a flowchart illustrating a flow of the processing of the coefficient calculation device 101 according to the first example embodiment.

First, the characteristic value calculation unit 102 calculates a characteristic value representing a character of each partial area represented by the partial image information 113 (Step S101). The characteristic value calculation unit 102 calculates, as the characteristic value, an area ratio of an area where plant leaves are present in the partial area (hereinafter, referred to as "leaf area").

Processing of calculating the characteristic value will be described in detail with reference to the characteristic value being the area ratio of the leaf area.

In the partial area, the characteristic value calculation unit 102 specifies a leaf area where plant leaves are present in the field 112. For example, the characteristic value calculation unit 102 specifies the leaf area based on an image in which the field 112 is taken by using a near infrared camera. When the image is a color image, the characteristic value calculation unit 102 may determine a certain area as the leaf area, for example, when a color in the certain area is within a range of a color representing the leaves.

The characteristic value calculation unit 102 calculates, as a characteristic value, an area ratio (hereinafter, referred to as a "coverage") of the leaf area over an area of the partial area. The ratio may not always be a mathematically defined ratio, and it is sufficient if the ratio represents a degree at which the leaf area occupies the partial area. The area ratio (i.e., the coverage) of the leaf area is an example of information having a high relevance with the extinction coefficient.

The characteristic value calculation unit 102 executes the above-mentioned processing for each of the partial areas and, thereby, calculates a coverage for the partial area as a characteristic value for the partial area. The characteristic value calculation unit 102 inputs the calculated characteristic value to the relevance information generation unit 105.

For a part of the partial areas among the plurality of partial areas, the first coefficient calculation unit 103 receives a vegetation index for the part of the partial areas from a sensor or the like mounted on an artificial satellite. The first coefficient calculation unit 103 receives a leaf area index for the part of the partial areas from the simulation unit 107.

The vegetation index is, for example, a normalized difference vegetation index (NDVI) or a weighted difference vegetation index (WDVI), which represents a distribution state of vegetation or activity of the vegetation in a certain area. NDVI is an abbreviation of normalized difference vegetation index. WDVI is an abbreviation of weighted difference vegetation index. NDVI is calculated, for example, in accordance with processing shown in Eqn. 1 based on a reflectance R of red in the visible region and a reflectance IR of a ray in the near infrared region by a sensor or the like mounted on an artificial satellite. Herein, the reflectances R and IR are observed for the vicinity of the field 112.

$$NDVI = (IR\text{-}R)/(IR\text{+}R) \quad ...(Eqn. \ 1)$$

Herein, a larger positive value of the NDVI represents a denser vegetation.

Meanwhile, WDVI can be calculated, for example, by applying processing shown in Eqn. 2 to a reflectance IR of a ray in the near infrared region and a reflectance R of red in the visible region. Herein, the reflectances IR and R are measured in accordance with the near infrared (NIR).

$$WDVI = IR\text{-}C{\times}R \quad ...(Eqn. \ 2)$$

Herein, C denotes a ratio of a reflectance SIR of the ray in the near infrared region when no plant is present in a partial area and a reflectance SR when no plant is present in the partial area.

For example, the leaf area index represents a leaf area index (LAI) included in a simulation model for rice weather relations (SIMRIW) or the like. SIMRIW is an abbreviation of simulation model for rice weather relations. LAI is an abbreviation of a leaf area index. The leaf area index LAI can be calculated, for example, by applying predetermined processing F shown in Eqn. 3 to an air temperature Te, a rainfall r, a time of sunshine ts, an amount of nitrogen Ni absorbable and present in a soil, and an irrigation amount w in a certain partial area.

$$LAI = F(Te, \ r, \ ts, \ Ni, \ w) \quad ...(Eqn. \ 3)$$

Eqn. 3 represents processing of calculating the leaf area index LAI based on a model capable of predicting a leaf area index based on the farming implemented in the field. For example, the model is information such as a partial differential equation including a parameter of the leaf area index, the information representing a relevance between the leaf area index and other pieces of information. The partial differential equation may not be always a single equation but may be a plurality of equations. For example, the model is discretized in accordance with a discretization method such as a finite element method. As a result, simultaneous linear equations are generated. For example, the predetermined processing F conceptually represents a processing procedure of calculating a solution of the simultaneous linear equations in accordance with a solution obtaining procedure such as an iterative method.

In accordance with such a procedure of calculating the leaf area index (LAI) as illustrated in Eqn. 3, for example, the simulation unit 107 simulates the farming implemented in the field 112. The simulation unit 107 may calculate the leaf area index, for example, by simulating a change of the leaf area index when a time elapses.

The first coefficient calculation unit 103 calculates the extinction coefficient for a part of the partial areas by processing the vegetation index for a part of the partial areas and the leaf area index (LAI) in accordance with predetermined calculation processing (illustrated in Eqn. 4) (Step S102). The first coefficient calculation unit 103 calculates an extinction coefficient $\alpha$ for the part of the partial areas, for example, in accordance with the processing illustrated in Eqn. 4.

$$\alpha = \text{-}1/LAI{\times}ln(1\text{-}WDVI/WDVII) \ ...(Eqn. \ 4)$$

Herein, WDVII denotes a limiting value of the WDVI. ln() denotes a logarithmic function having a Napier's constant as a base.

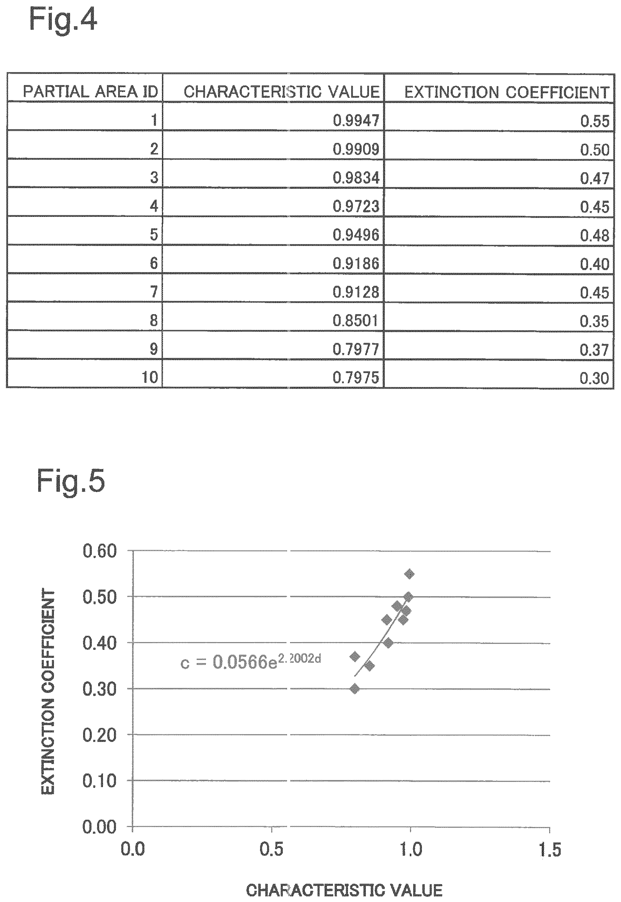

The relevance information generation unit 105 receives the extinction coefficient for a certain part of the partial areas from the first coefficient calculation unit 103, and further, receives the characteristic value (for example, a coverage (i.e., a ratio of the area where leaves are present)) for the certain part of the partial areas from the characteristic value calculation unit 102. The relevance information generation unit 105 generates information, for example, as illustrated in Fig. 4, in which a characteristic value for a certain part of the partial areas and an extinction coefficient for the certain part of the partial areas are associated with each other. Fig. 4 is a diagram conceptually illustrating an example of the information generated by the relevance information generation unit 105.

In the information illustrated in Fig. 4, an identifier (ID) for identifying each partial area, the characteristic value for the partial area and the extinction coefficient for the partial area are associated with one another. This represents information for the characteristic value related to each partial area identified by the partial area ID and the extinction coefficient related to the partial area. For example, the partial area ID "3", the characteristic value "0.9834", and the extinction coefficient "0.47" are associated with one another. This represents that the characteristic value for a partial area identified by the partial area ID "3" is "0.9834", and that the extinction coefficient for the partial area is "0.47",

In the description below, for convenience of explanation, a set of the extinction coefficient for the partial area and the characteristic value for the partial area is referred to as a "set". In this case, the relevance information generation unit 105 generates the set for the partial area.

The relevance information generation unit 105 generates relevance information representing a relevance between an extinction coefficient and a characteristic value (Step S103). The relevance information generation unit 105 calculates the relevance information by obtaining a function that fits to a relevance between the extinction coefficient and the characteristic value (for example, a coverage), for example, as illustrated in Fig. 5. Fig. 5 is a diagram conceptually illustrating an example of the calculated relevance information. The function is a function such as an exponential function and a polynomial function. A horizontal axis in Fig. 5 represents the characteristic value, with an increasing value toward the right side. A vertical axis in Fig. 5 represents the extinction coefficient, with an increasing value toward an upper side. In a case of the example shown in Fig. 5, the relevance information generation unit 105 calculates, as the relevance information, "extinction coefficient c = 0.0566 × exp(2.2002 × characteristic value d)" (where exp() denotes an exponential function having a Napier's constant e as a base).

The second coefficient calculation unit 104 receives the relevance information generated by the relevance information generation unit 105, and a characteristic value (for example, a coverage) for a partial area different from the above-mentioned part of the partial areas (i.e., a partial area for which an extinction coefficient is not calculated by the first coefficient calculation unit 103) among the plurality of partial areas. The second coefficient calculation unit 104 calculates an extinction coefficient for the different partial area by calculating an extinction coefficient corresponding to the characteristic value in the relevance information (Step S104).

The index calculation unit 106 receives the extinction coefficient from the second coefficient calculation unit 104. The index calculation unit 106 calculates a leaf area index (LAI) for the partial area by applying processing shown in Eqn. 5 to the extinction coefficient for the partial area and a vegetation index (WDVI) for the partial area.

$$LAI = -1/\alpha \times \ln(1 - WDVI/WDVII) \quad ...(Eqn.\ 5)$$

Next, an advantageous effect of the coefficient calculation device 101 according to the first example embodiment of the present invention will be described.

In accordance with the coefficient calculation device 101 according to the first example embodiment, an accurate extinction coefficient for a certain area can be calculated in a short period. A reason for this is that, in the coefficient calculation device 101, the predetermined calculation processing is executed only for a part of the partial areas in the field area, and the extinction coefficient is calculated for a partial area different from the part of the partial areas, based on the characteristic value of the partial area. Moreover, a workload in the processing of calculating the extinction coefficient based on the characteristic value (mentioned above while calculating Fig. 5), is smaller than a workload in the processing of calculating the extinction coefficient in accordance with the predetermined calculation processing (mentioned above with reference to Eqn. 3). Hence, even when a long processing time is required for the predetermined calculation processing, then in accordance with the coefficient calculation device 101, accurate extinction coefficients for a plurality of the partial areas in the field area can be calculated in a short period.

Moreover, in accordance with the coefficient calculation device 101 according to the first example embodiment, the extinction coefficients for the plurality of partial areas in the field area can be calculated more accurately. A reason for this is that the coefficient calculation device 101 calculates the extinction coefficient based on the area ratio of the leaf area having a high relevance with the extinction coefficient. In other words, since the area ratio of the leaf area has a high relevance with the extinction coefficient, the extinction coefficient calculated by the coefficient calculation device 101 is a more accurate value.

Moreover, in accordance with the coefficient calculation device 101 according to the first example embodiment, an accurate leaf area index (LAI) can be calculated with a small workload. A reason for this is that the processing of calculating the leaf area index in accordance with Eqn. 5 requires a smaller workload than the processing of calculating the leaf area index in accordance with Eqn. 3, and moreover, as mentioned above, the extinction coefficient as a base of calculating the leaf area index is an accurate value.

<Second example embodiment>

Next, a second example embodiment of the present invention, which is based on the above-mentioned first example embodiment, will be described.

In the description below, characteristic portions according to this example embodiment will be mainly described, and the same reference numerals will be assigned to similar components to those of the above-mentioned first example embodiment, whereby a repeated description will be omitted.

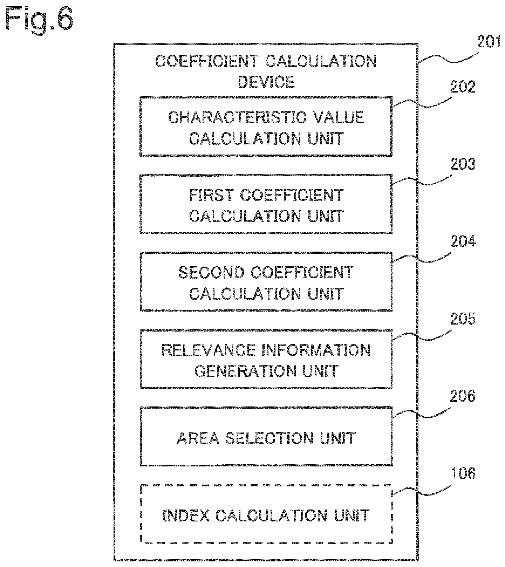

Referring to Fig. 6, a configuration of a coefficient calculation device 201 according to a second example embodiment of the present invention will be described in detail. Fig. 6 is a block diagram illustrating the configuration of the coefficient calculation device 201 according to the second example embodiment of the present invention.

The coefficient calculation device 201 according to the second example embodiment includes a characteristic value calculation unit (characteristic value calculator) 202, a first coefficient calculation unit (first coefficient calculator) 203, a second coefficient calculation unit (second coefficient calculator) 204, a relevance information generation unit (relevance information generator) 205, and an area selection unit (area selector) 206. The coefficient calculation device 201 may further include an index calculation unit (index calculator) 106.

The first coefficient calculation unit 203 has a configuration similar to the configuration of the first coefficient calculation unit 103 (Fig. 1). However, for a part of the partial areas, which is selected from among a plurality of the partial areas by the area selection unit 206, the first coefficient calculation unit 203 calculates an extinction coefficient in accordance with predetermined calculation processing. The second coefficient calculation unit 204 has a configuration similar to the configuration of the second coefficient calculation unit 104 (Fig. 1). The relevance information generation unit 205 has a configuration similar to the configuration of the relevance information generation unit 105 (Fig. 1).

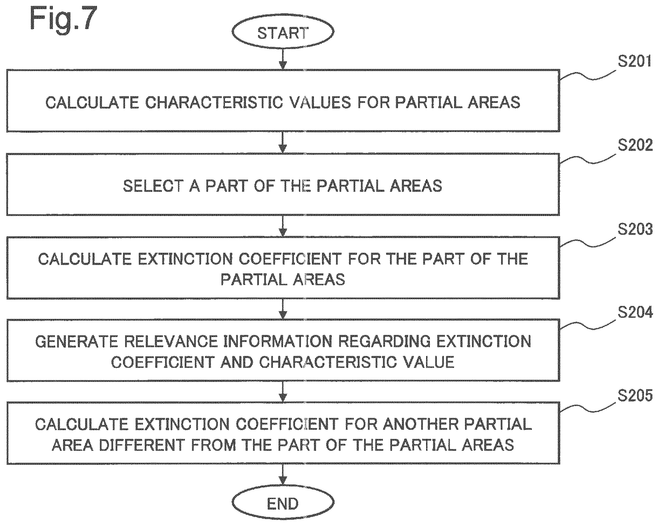

Next, referring to Fig. 7, processing of the coefficient calculation device 201 according to the second example embodiment of the present invention will be described in detail. Fig. 7 is a flowchart illustrating a flow of the processing of the coefficient calculation device 201 according to the second example embodiment.

The characteristic value calculation unit 202 calculates a characteristic value representing a character for each partial area represented by the partial image information 113 (illustrated in Fig. 3) (Step S201). Processing of Step S201 is processing similar to the processing of Step S101 (Fig. 2).

From among a plurality of the partial areas in the field area, the area selection unit 206 selects, as a part of the partial areas, a partial area that satisfies a predetermined selection condition (Step S202). As mentioned above, for the part of the partial areas selected by the area selection unit 206, the first coefficient calculation unit 203 calculates the extinction coefficient in accordance with the processing as illustrated in Eqn. 4. The processing by which the area selection unit 206 selects the part of the partial areas will be specifically described.

The processing will be described with reference to an example of the case where the predetermined selection condition is a condition for position information representing a position of the partial area. For example, the predetermined selection condition is a condition that a scatter degree (a degree of scattering) of position information representing positions of the partial areas is larger than a predetermined scatter degree. For example, the scatter degree is dispersion for the positions of the partial areas, and is a larger value as the partial areas are scattered in the field 112 (Fig. 3). The scatter degree may be a numeric value similar to that of the dispersion. The area selection unit 206 receives the partial image information 113 (illustrated in Fig. 3) representing each partial area and the position information representing the position of the partial area from a sensor, a storage device, or the like. From among a plurality of partial areas, the area selection unit 206 selects a partial area that satisfies the condition that the scatter degree of the position information is larger than a predetermined scatter degree. In other words, from among a plurality of partial areas, the area selection unit 206 selects, as a part of the partial areas, a partial area that satisfies a condition that the partial area positionally varies.

The processing executed by the area selection unit 206 will be described with reference to an example of the case where the predetermined selection condition is a condition for the characteristic value of the partial area (for example, the area ratio of the leaf area). For example, the predetermined selection condition is a condition that a scatter degree of characteristic values for the partial areas is larger than a predetermined scatter degree. In this case, the area selection unit 206 receives the partial image information 113 (illustrated in Fig. 3), which represents each partial area, from a sensor, a storage device, or the like, and receives a characteristic value, which is calculated for the partial area, from the characteristic value calculation unit 202. From among a plurality of partial areas, the area selection unit 206 selects a partial area that satisfies the condition that the scatter degree of the characteristic values is larger than the predetermined scatter degree. In other words, from among a plurality of partial areas, the area selection unit 206 selects, as a part of the partial areas, a partial area that satisfies a condition that the characteristic value for the partial area varies.

Next, for the part of the partial areas selected by the area selection unit 206, the first coefficient calculation unit 203 calculates an extinction coefficient in accordance with predetermined calculation processing (Step S203). Processing of Step S203 is processing similar to the processing of Step S102 (Fig. 2).

Thereafter, processing similar to those in Step S103 and Step S104 in Fig. 2 is executed (Step S204 and Step S205).

Next, an advantageous effect regarding the coefficient calculation device 201 according to the second example embodiment of the present invention will be described.

In accordance with the coefficient calculation device 201 according to the second example embodiment, an accurate extinction coefficient for a certain area can be calculated in a short period. A reason for this is similar to the reason described in the first example embodiment.

In accordance with the coefficient calculation device 201 according to the second example embodiment, the extinction coefficients for the plurality of partial areas in the field area can be calculated more accurately. A reason for this is that the partial area selected by the area selection unit 206 is suitable for generating the relevance information representing the relevance between the extinction coefficient and the characteristic value.

When the area selection unit 206 selects the partial area that satisfies the condition that the scatter degree of the characteristic values is larger than the predetermined scatter degree, the relevance information generation unit 205 generates the relevance information based on the characteristic values scattered in a wide range. Hence, the characteristic values are not biased, and accordingly, the relevance information calculated based on the characteristic values represents a more accurate relevance.

Moreover, when the area selection unit 206 selects the partial areas which satisfy the condition that the scatter degree of the position information is larger than the predetermined scatter degree, there is a high possibility that the characteristic values for the partial areas are scattered in a wider range in terms of the scatter degree. As a result, the relevance information generation unit 205 generates the relevance information based on the characteristic values scattered in the wide range. Hence, the characteristic values are not biased, and accordingly, there is a high possibility that the relevance information calculated based on the characteristic values represents a more accurate relevance.

<Third example embodiment>

Next, a third example embodiment of the present invention, which is based on the above-mentioned first example embodiment, will be described.

In the description below, characteristic portions according to this example embodiment will be mainly described, and the same reference numerals will be assigned to similar components to those of the above-mentioned first example embodiment, whereby a repeated description will be omitted.

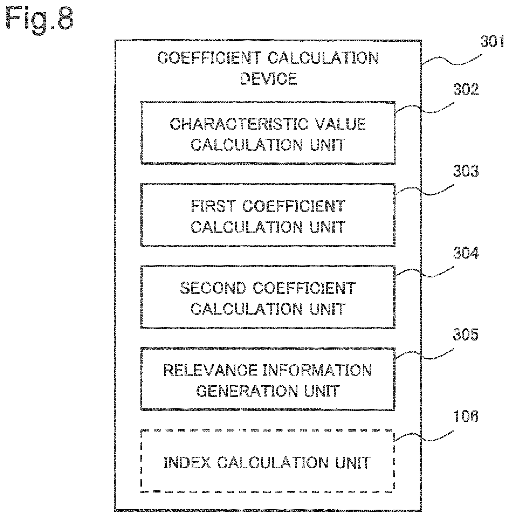

Referring to Fig. 8, a configuration of a coefficient calculation device 301 according to a third example embodiment of the present invention will be described in detail. Fig. 8 is a block diagram illustrating the configuration of the coefficient calculation device 301 according to the third example embodiment of the present invention.

The coefficient calculation device 301 according to the third example embodiment includes a characteristic value calculation unit (characteristic value calculator) 302, a first coefficient calculation unit (first coefficient calculator) 303, a second coefficient calculation unit (second coefficient calculator) 304, and a relevance information generation unit (relevance information generator) 305.

The first coefficient calculation unit 303 has a configuration similar to the configuration of the first coefficient calculation unit 103 (Fig. 1). The second coefficient calculation unit 304 has a configuration similar to the configuration of the second coefficient calculation unit 104 (Fig. 1).

The relevance information generation unit 305 receives the extinction coefficient calculated for a certain part of the partial areas from the first coefficient calculation unit 303. The relevance information generation unit 305 receives the characteristic value calculated for the certain part of the partial areas (for example, the area ratio of the leaf area) from the characteristic value calculation unit 302. The relevance information generation unit 305 receives position information, which represents a position of the certain part of the partial areas, from a sensor, a storage device, or the like. The relevance information generation unit 305 generates information (illustrated in Fig. 4) in which the extinction coefficient for the certain part of the partial areas and the characteristic value for the certain part of the partial areas are associated with each other. The relevance information generation unit 305 may further generate information in which a partial area ID for identifying the certain part of the partial areas is associated with the extinction coefficient for the certain part of the partial areas.

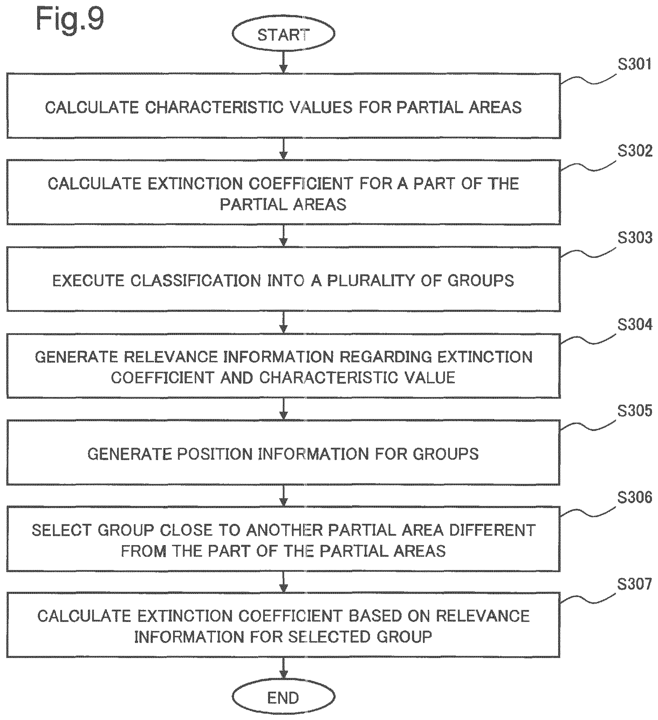

Next, referring to Fig. 9, processing of the coefficient calculation device 301 according to the third example embodiment of the present invention will be described in detail. Fig. 9 is a flowchart illustrating a flow of the processing of the coefficient calculation device 301 according to the third example embodiment.

First, processing similar to the processing illustrated in Step S101 and Step S102 (Fig. 2) is executed (Step S301 and Step S302).

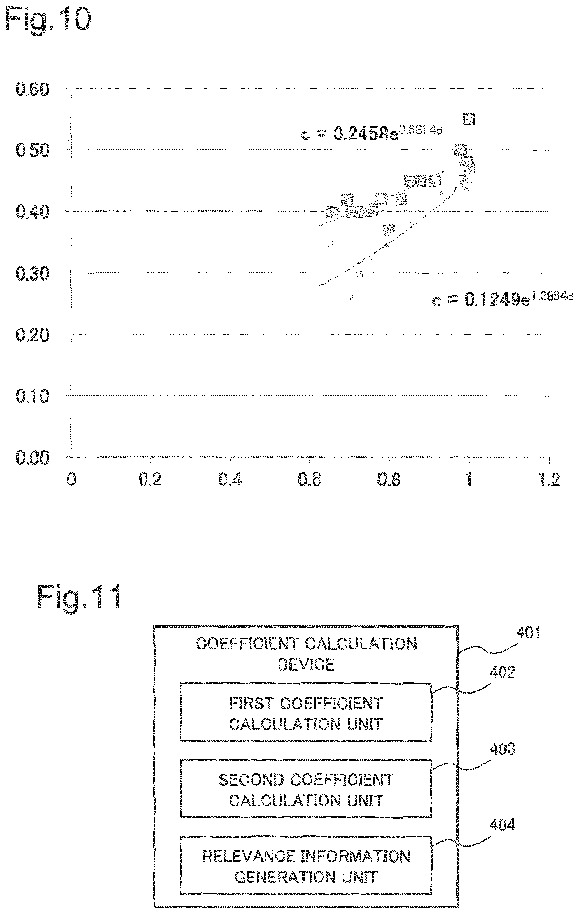

The relevance information generation unit 305 classifies such sets (illustrated in Fig. 4) in each of which the characteristic value and the extinction coefficient are associated with each other into a plurality of groups similar to one another (Step S303). The relevance information generation unit 305 classifies the sets into the plurality of groups, for example, in accordance with a clustering method. As illustrated in Fig. 10, for each of the groups, the relevance information generation unit 305 generates relevance information representing a relevance based on the sets belonging to the group (Step S304), and further, generates position information representing a position for the group (for example, an average of positions of the partial areas belonging to the group) (Step S305). Fig. 10 is a diagram conceptually illustrating an example of the relevance information calculated for each group.

A horizontal axis in Fig. 10 represents the characteristic value, with an increasing value toward the right side. A vertical axis in Fig. 10 represents the extinction coefficient, with an increasing value toward the upper side. In a case of the example shown in Fig. 10, the sets are classified into a first group shown by rectangles and a second group shown by triangles. The relevance information generation unit 305 calculates, as relevance information for the first group, "extinction coefficient c = 0.2458×exp(0.6814 × characteristic value d)" (where exp() denotes an exponential function having a Napier's constant as a base). The relevance information generation unit 305 calculates "extinction coefficient c = 0.1249×exp(1.2864 × characteristic value d)" as relevance information for the second group.

The relevance information generation unit 305 may execute processing of order of Step S305 and Step S304.

The second coefficient calculation unit 304 receives relevance information for each group and position information for the group from the relevance information generation unit 305. The second coefficient calculation unit 304 receives position information representing a position of a partial area different from the certain part of the partial areas, from a sensor, a storage device, or the like. The second coefficient calculation unit 304 receives a characteristic value for the different partial area from the characteristic value calculation unit 302. The second coefficient calculation unit 304 selects a group (hereinafter, referred to as "selection group") positionally closest to the position of the different partial area, based on position information for the group (Step S306). The second coefficient calculation unit 304 calculates an extinction coefficient for the different partial area, based on the relevance information generated for the selection group and the characteristic value for the different partial area (Step S307).

Next, a description will be given of an advantageous effect regarding the coefficient calculation device 301 according to the third example embodiment of the present invention.

In accordance with the coefficient calculation device 301 according to the third example embodiment, an accurate extinction coefficient for a certain area can be calculated in a short period. A reason for this is similar to the reason described in the first example embodiment.

In accordance with the coefficient calculation device 301 according to the third example embodiment, the extinction coefficients for the plurality of partial areas in the field area can be calculated more accurately. A reason for this will be described. Frequently, the extinction coefficient gently changes between adjacent partial areas. Therefore, there is a high possibility that the relevance information calculated based on the partial area included in the group close in terms of distance accurately represents relevance information for a partial area close to the partial area. Hence, the coefficient calculation device 301 calculates an extinction coefficient in a partial area to be a target, based on relevance information for a partial area close to such a target partial area, and can thereby calculate the extinction coefficient accurately.

<Fourth example embodiment>

Next, a fourth example embodiment of the present invention will be described.

Referring to Fig. 11, a configuration of a coefficient calculation device 401 according to a fourth example embodiment of the present invention will be described in detail. Fig. 11 is a block diagram illustrating the configuration of the coefficient calculation device 401 according to the fourth example embodiment of the present invention.

The coefficient calculation device 401 according to the fourth example embodiment includes a first coefficient calculation unit (first coefficient calculator) 402, a second coefficient calculation unit (second coefficient calculator) 403, and a relevance information generation unit (relevance information generator) 404.

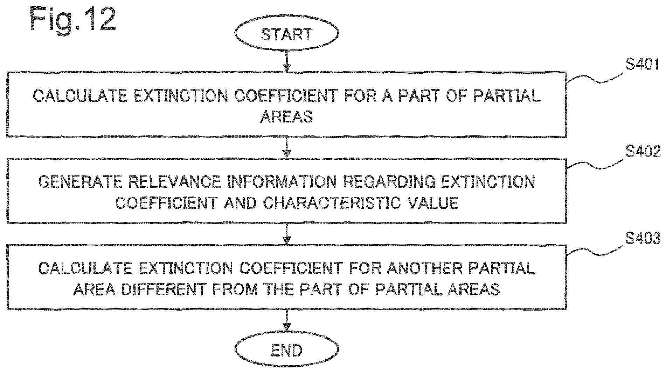

Next, referring to Fig. 12, processing of the coefficient calculation device 401 according to the fourth example embodiment of the present invention will be described in detail. Fig. 12 is a flowchart illustrating a flow of the processing of the coefficient calculation device 401 according to the fourth example embodiment.

In accordance with predetermined calculation processing, the first coefficient calculation unit 402 calculates an extinction coefficient for a part of partial areas among a plurality of partial areas included in the field 112 (Fig. 3) (Step S401). For example, the predetermined calculation processing is processing of calculating a leaf area index based on an amount of irrigation implemented in the part of the partial areas, executing the processing illustrated in Eqn. 4 for a vegetation index (for example, NDVI, WDVI) for the part of the partial areas and for the calculated leaf area index, and, thereby, calculating the extinction coefficient for the part of the partial areas.

The relevance information generation unit 404 receives the extinction coefficient for the part of the partial areas from the first coefficient calculation unit 402. The relevance information generation unit 404 receives a characteristic value representing a character for the part of the partial areas from a sensor, a storage device, or the like. For example, the characteristic value is a coverage representing an area ratio of a leaf area where leaves are present in the part of the partial areas. The relevance information generation unit 404 generates relevance information (for example, Fig. 5 and Fig. 10) representing a relevance between the extinction coefficient and the characteristic value. For example, the relevance information generation unit 404 generates the relevance information by calculating a function that fits to a relevance between the extinction coefficient and the characteristic value (Step S402).

The second coefficient calculation unit 403 receives the relevance information from the relevance information generation unit 404. The second coefficient calculation unit 403 receives a characteristic value for a partial area different from the part of the partial areas from a sensor, a storage device, or the like. The second coefficient calculation unit 403 calculates an extinction coefficient for the different partial area by calculating an extinction coefficient corresponding to the characteristic value, based on the relevance information (Step S403).

The first coefficient calculation unit 402 can be achieved by using a function similar to a function included in the first coefficient calculation unit 103 (Fig. 1) according to the first example embodiment, a function included in the first coefficient calculation unit 203 (Fig. 6) according to the second example embodiment, or a function included in the first coefficient calculation unit 303 (Fig. 8) according to the third example embodiment. The second coefficient calculation unit 403 can be achieved by using a function similar to a function included in the second coefficient calculation unit 104 (Fig. 1) according to the first example embodiment, a function included in the second coefficient calculation unit 204 (Fig. 6) according to the second example embodiment, or a function included in the second coefficient calculation unit 304 (Fig. 8) according to the third example embodiment. The relevance information generation unit 404 can be achieved by using a function similar to a function included in the relevance information generation unit 105 (Fig. 1) according to the first example embodiment, a function included in the relevance information generation unit 205 (Fig. 6) according to the second example embodiment, or a function included in the relevance information generation unit 305 (Fig. 8) according to the third example embodiment.

Hence, the coefficient calculation device 401 can be achieved by using a function similar to a function included in the coefficient calculation device 101 (Fig. 1) according to the first example embodiment, a function included in the coefficient calculation device 201 (Fig. 6) according to the second example embodiment, or a function included in the coefficient calculation device 301 (Fig. 8) according to the third example embodiment.

Next, an advantageous effect regarding the coefficient calculation device 401 according to the fourth example embodiment of the present invention will be described.

In accordance with the coefficient calculation device 401 according to the fourth example embodiment, an accurate extinction coefficient for a certain area can be calculated in a short period. A reason for this is that, in the coefficient calculation device 401, the predetermined calculation processing is executed only for a part of the partial areas in the field area, and the extinction coefficient is calculated for a partial area different from the part of the partial areas, based on the characteristic value of the partial area. Hence, even when a long processing time is required for the predetermined calculation processing, then in accordance with the coefficient calculation device 401, accurate extinction coefficients for a plurality of the partial areas in the field area can be calculated in a short period.

(Hardware Configuration Example)

A configuration example of hardware resources that achieve a coefficient calculation device according to each example embodiment of the present invention using a computer processing device (information processing device, compute) will be described. However, the coefficient calculation device may be achieved using physically or functionally at least two calculation processing devices. Further, the coefficient calculation device may be achieved as a dedicated device.

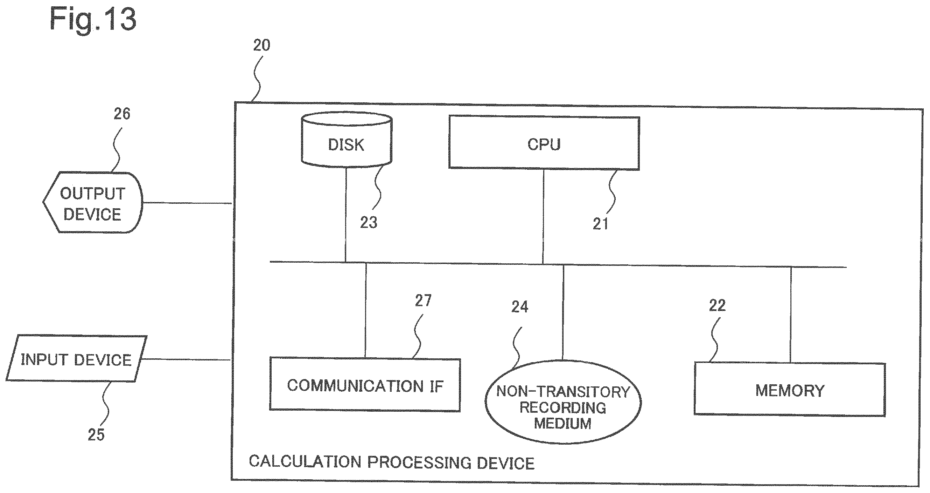

Fig. 13 is a block diagram schematically illustrating a hardware configuration of a calculation processing device capable of achieving a coefficient calculation device according to each example embodiment of the present invention. A calculation processing device 20 includes a central processing unit (hereinafter, referred to as "CPU") 21, a memory 22, a disk (disc) 23, a non-transitory recording medium 24, and a communication interface (hereinafter, referred to as "communication I/F") 27. The calculation processing device 20 may connect an input device 25 and an output device 26. The calculation processing device 20 can execute transmission/reception of information to/from another calculation processing device and a communication device via the communication I/F 27.

The non-transitory recording medium 24 is, for example, a computer-readable Compact Disc, Digital Versatile Disc. The non-transitory recording medium 24 may be Universal Serial Bus (USB) memory, Solid State Drive or the like. The non-transitory recording medium 24 allows a related program to be holdable and portable without power supply. The non-transitory recording medium 24 is not limited to the above-described media. Further, a related program can be carried via a communication network by way of the communication I/F 27 instead of the non-transitory recording medium 24.

In other words, the CPU 21 copies, on the memory 22, a software program (a computer program: hereinafter, referred to simply as a "program") stored in the disk 23 when executing the program and executes arithmetic processing. The CPU 21 reads data necessary for program execution from the memory 22. When display is needed, the CPU 21 displays an output result on the output device 26. When a program is input from the outside, the CPU 21 reads the program from the input device 25. The CPU 21 interprets and executes a coefficient calculation program (Fig. 2, Fig. 7, Fig. 9, or Fig.

12) present on the memory 22 corresponding to a function (processing) indicated by each unit illustrated in Fig. 1, Fig. 6, Fig. 8, or Fig. 11 described above. The CPU 21 sequentially executes the processing described in each example embodiment of the present invention.

In other words, in such a case, it is conceivable that the present invention can also be made using the coefficient calculation program. Further, it is conceivable that the present invention can also be made using a computer-readable, non-transitory recording medium storing the coefficient calculation program.

The present invention has been described using the above-described example embodiments as example cases. However, the present invention is not limited to the above-described example embodiments. In other words, the present invention is applicable with various aspects that can be understood by those skilled in the art without departing from the scope of the appended claims.

[Reference signs List]

| 101 | coefficient calculation device |
| 102 | characteristic value calculation unit |
| 103 | first coefficient calculation unit |
| 104 | second coefficient calculation unit |
| 105 | relevance information generation unit |
| 106 | index calculation unit |
| 107 | simulation unit |
| 111 | image information |
| 112 | field |
| 113 | partial image information |
| 201 | coefficient calculation device |
| 202 | characteristic value calculation unit |
| 203 | first coefficient calculation unit |
| 204 | second coefficient calculation unit |
| 205 | relevance information generation unit |
| 206 | area selection unit |
| 301 | coefficient calculation device |
| 302 | characteristic value calculation unit |
| 303 | first coefficient calculation unit |
| 304 | second coefficient calculation unit |
| 305 | relevance information generation unit |
| 401 | coefficient calculation device |
| 402 | first coefficient calculation unit |
| 403 | second coefficient calculation unit |
| 404 | relevance information generation unit |
| 20 | calculation processing device |
| 21 | CPU |
| 22 | memory |
| 23 | disk |
| 24 | non-transitory recording medium |
| 25 | input device |
| 26 | output device |
| 27 | communication IF |

## Claims

1. An extinction coefficient calculation device comprising:

means for receiving an image (111) taken in accordance with an angle view of a field (112);
first coefficient calculation means (103; 203; 303) for calculating an extinction coefficient for a part of partial areas, among a plurality of partial areas (113) of the received image (111) in accordance with a predetermined calculation processing;
characteristic value calculation means (102; 202; 302) for calculating, as a characteristic value, an area ratio of leaf area in each of the partial areas (113) using the received image (111);

relevance information generation means (105; 205: 305) for generating a function between the extinction coefficient calculated for the part of partial areas (113) and the characteristic value calculated for the part of partial areas (113), the function being an exponential function or a polynomial function; and

second coefficient calculation means (104; 204; 304) for calculating an extinction coefficient for another partial area by applying the characteristic value calculated for the another partial area to the generated function, the another partial area being different from the part of partial areas,

wherein the first coefficient calculation means (103; 203; 303) is configured to obtain a vegetation index for the part of partial areas using the received image (111), and apply the predetermined calculation processing to the obtained vegetation index and a leaf area index for the part of partial areas, thereby calculating the extinction coefficient for the part of partial areas.

2. The coefficient calculation device according to claim 1, further comprising:
field selection means (206) for selecting, as the part of partial areas, partial areas with having a scatter degree of position information larger than a predetermined scatter degree from the plurality of partial areas, the position information representing positions of the partial areas.

3. The coefficient calculation device according to claim 1, further comprising:
field selection means (206) for selecting, as the part of partial areas, partial areas with having a scatter degree of the characteristic values larger than a predetermined scatter degree from the plurality of partial areas.

4. The coefficient calculation device according to any one of claims 1 to 3, wherein

the relevance information generation means (305) is configured to
classify the pairs of extinction coefficient/characteristic value for the respective partial areas into a plurality of groups in accordance with a clustering method,
generate the function for each of the classified groups, and
generate position information that indicates an average position of positions of partial areas with respect to the pairs of extinction coefficient/characteristic value belonging to each group for each group, based on position information indicating positions of said partial areas, and
the second coefficient calculation means (304) is configured to
select a group, and
calculate the extinction coefficient for the another partial area based on the function generated for the selected group,
the selected group being positionally closest to the another partial area among the plurality of groups.

5. The coefficient calculation device according to any one of claims 1 to 4, further comprising:
simulation means (107) for calculating the leaf area index based on an amount of irrigation in the field.

6. The coefficient calculation device according to any one of claims 1 to 4, further comprising:
index calculation means (106) for calculating a leaf area index for the another partial area based on the extinction coefficient calculated by the second coefficient calculation means (104; 204; 304) for the another partial area and a vegetation index for the another partial area.

7. An extinction coefficient calculation method by an information processing device comprising:

receiving an image (111) taken in accordance with an angle view of a field (112)
calculating an extinction coefficient for a part of partial areas, among a plurality of partial areas (113) of the received image (111) in accordance with a predetermined calculation processing;
calculating, as a characteristic value, an area ratio of leaf area in each of the partial areas (113) using the received image (111);
generating a function between the extinction coefficient calculated for the part of partial areas (113) and the characteristic value calculated for the part of partial areas (113), the function being an exponential function or a polynomial function; and
calculating an extinction coefficient for the another partial area by applying the characteristic value calculated for another partial area to the generated function, the another partial area being different from the part of partial areas,
wherein the step of calculating an extinction coefficient for the part of partial areas comprises:
obtaining a vegetation index for the part of partial areas using the received image (111), and applying the predetermined calculation processing to the obtained vegetation index and a leaf area index for the part of partial areas, thereby calculating the extinction coefficient for the part of partial areas.

8. A coefficient calculation program causing a compute to execute the method according to claim 7.

**Patentansprüche**

1. Extinktionskoeffizienten-Berechnungsvorrichtung, aufweisend:

   Mittel zum Empfangen eines Bildes (111), das in Übereinstimmung mit einem Bildwinkel eines Feldes (112) aufgenommen wurde;
   erste Koeffizientenberechnungsmittel (103; 203; 303) zum Berechnen eines Extinktionskoeffizienten für einen Teil von Teilbereichen aus einer Mehrzahl von Teilbereichen (113) des empfangenen Bildes (111) in Übereinstimmung mit einer vorbestimmten Berechnungsverarbeitung;
   Merkmalswertberechnungsmittel (102; 202; 302) zum Berechnen, als einen Merkmalswert, eines Flächenverhältnisses der Blattfläche in jedem der Teilbereiche (113) unter Verwendung des empfangenen Bildes (111);
   Relevanzinformationserzeugungsmittel (105; 205; 305) zum Erzeugen einer Funktion zwischen dem für den Teil der Teilbereiche (113) berechneten Extinktionskoeffizienten und dem für den Teil der Teilbereiche (113) berechneten Merkmalswert, wobei die Funktion eine Exponentialfunktion oder eine Polynomfunktion ist; und
   zweite Koeffizientenberechnungsmittel (104; 204; 304) zum Berechnen eines Extinktionskoeffizienten für einen anderen Teilbereich durch Anwenden des für den anderen Teilbereich berechneten Merkmalswerts auf die erzeugte Funktion, wobei sich der andere Teilbereich von dem Teil der Teilbereiche unterscheidet,
   wobei die ersten Koeffizientenberechnungsmittel (103; 203; 303) konfiguriert sind, um einen Vegetationsindex für den Teil der Teilbereiche unter Verwendung des empfangenen Bildes (111) zu erhalten und die vorbestimmte Berechnungsverarbeitung auf den erhaltenen Vegetationsindex und einen Blattflächenindex für den Teil der Teilbereiche anzuwenden, wodurch der Extinktionskoeffizient für den Teil der Teilbereiche berechnet wird.

2. Koeffizientenberechnungsvorrichtung nach Anspruch 1, ferner aufweisend:
   Feldauswahlmittel (206) zum Auswählen, als den Teil von Teilbereichen, von Teilbereichen, die einen Streuungsgrad von Positionsinformationen aufweisen, der größer als ein vorbestimmter Streuungsgrad ist, aus der Mehrzahl von Teilbereichen, wobei die Positionsinformationen Positionen der Teilbereiche repräsentieren.

3. Koeffizientenberechnungsvorrichtung nach Anspruch 1, ferner aufweisend:
   Feldauswahlmittel (206) zum Auswählen, als den Teil von Teilbereichen, von Teilbereichen, die einen Streuungsgrad der Merkmalswerte aufweisen, der größer als ein vorbestimmter Streuungsgrad ist, aus der Mehrzahl von Teilbereichen.

4. Koeffizientenberechnungsvorrichtung nach einem der Ansprüche 1 bis 3, wobei die Relevanzinformationserzeugungsmittel (305) konfiguriert sind, zum Klassifizieren der Paare von Extinktionskoeffizient/Merkmalswert für die jeweiligen Teilbereiche in eine Mehrzahl von Gruppen in Übereinstimmung mit einem Clustering-Verfahren,

   Erzeugen der Funktion für jede der klassifizierten Gruppen, und
   Erzeugen von Positionsinformationen, die eine Durchschnittsposition von Positionen von Teilbereichen in Bezug auf die Paare von Extinktionskoeffizient/Merkmalswert, die zu jeder Gruppe gehören, für jede Gruppe angeben, basierend auf Positionsinformationen, die Positionen der Teilbereiche angeben, und
   die zweiten Koeffizientenberechnungsmittel (304) konfiguriert sind zum Auswählen einer Gruppe, und
   Berechnen des Extinktionskoeffizienten für den anderen Teilbereich basierend auf der für die ausgewählte Gruppe erzeugten Funktion,
   wobei die ausgewählte Gruppe unter der Mehrzahl von Gruppen dem anderen Teilbereich örtlich am nächsten ist.

5. Koeffizientenberechnungsvorrichtung nach einem der Ansprüche 1 bis 4, ferner aufweisend:
   Simulationsmittel (107) zum Berechnen des Blattflächenindex basierend auf einer Bewässerungsmenge in dem Feld.

6. Koeffizientenberechnungsvorrichtung nach einem der Ansprüche 1 bis 4, ferner aufweisend:
   Indexberechnungsmittel (106) zum Berechnen eines Blattflächenindex für den anderen Teilbereich basierend auf dem durch die zweiten Koeffizientenberechnungsmittel (104; 204; 304) berechneten Extinktionskoeffizienten für den anderen Teilbereich und einem Vegetationsindex für den anderen Teilbereich.

7. Extinktionskoeffizienten-Berechnungsverfahren durch eine Informationsverarbeitungsvorrichtung, umfassend:

Empfangen eines Bildes (111), das in Übereinstimmung mit einem Bildwinkel eines Feldes (112) aufgenommen wurde;

Berechnen eines Extinktionskoeffizienten für einen Teil von Teilbereichen aus einer Mehrzahl von Teilbereichen (113) des empfangenen Bildes (111) in Übereinstimmung mit einer vorbestimmten Berechnungsverarbeitung;

Berechnen, als einen Merkmalswert, eines Flächenverhältnisses der Blattfläche in jedem der Teilbereiche (113) unter Verwendung des empfangenen Bildes (111);

Erzeugen einer Funktion zwischen dem für den Teil der Teilbereiche (113) berechneten Extinktionskoeffizienten und dem für den Teil der Teilbereiche (113) berechneten Merkmalswert, wobei die Funktion eine Exponentialfunktion oder eine Polynomfunktion ist; und

Berechnen eines Extinktionskoeffizienten für den anderen Teilbereich durch Anwenden des für einen anderen Teilbereich berechneten Merkmalswerts auf die erzeugte Funktion, wobei sich der andere Teilbereich von dem Teil der Teilbereiche unterscheidet,

wobei der Schritt des Berechnens eines Extinktionskoeffizienten für den Teil der Teilbereiche umfasst:

Erhalten eines Vegetationsindex für den Teil der Teilbereiche unter Verwendung des empfangenen Bildes (111) und Anwenden der vorbestimmten Berechnungsverarbeitung auf den erhaltenen Vegetationsindex und einen Blattflächenindex für den Teil der Teilbereiche, wodurch der Extinktionskoeffizient für den Teil der Teilbereiche berechnet wird.

8. Koeffizientenberechnungsprogramm, das einen Computer veranlasst, das Verfahren nach Anspruch 7 auszuführen.

**Revendications**

1. Dispositif de calcul de coefficient d'extinction comportant :

un moyen pour recevoir une image (111) prise conformément à une vue angulaire d'un champ (112) ;
un premier moyen de calcul de coefficient (103 ; 203 ; 303) pour calculer un coefficient d'extinction pour une partie de zones partielles, parmi une pluralité de zones partielles (113) de l'image reçue (111) conformément à un traitement de calcul prédéterminé ;
un moyen de calcul de valeur caractéristique (102 ; 202 ; 302) pour calculer, en tant que valeur caractéristique, un rapport de zone de zone de feuilles dans chacune des zones partielles (113) à l'aide de l'image reçue (111) ;
un moyen de génération d'informations de pertinence (105 ; 205 ; 305) pour générer une fonction entre le coefficient d'extinction calculé pour la partie de zones partielles (113) et la valeur caractéristique calculée pour la partie de zones partielles (113), la fonction étant une fonction exponentielle ou une fonction polynomiale ; et
un second moyen de calcul de coefficient (104 ; 204 ; 304) pour calculer un coefficient d'extinction pour une autre zone partielle en appliquant la valeur caractéristique calculée pour l'autre zone partielle à la fonction générée, l'autre zone partielle étant différente de la partie de zones partielles,
dans lequel le premier moyen de calcul de coefficient (103 ; 203 ; 303) est configuré pour obtenir un indice de végétation pour la partie de zones partielles à l'aide de l'image reçue (111), et appliquer le traitement de calcul prédéterminé à l'indice de végétation obtenu et à un indice de zone de feuilles pour la partie de zones partielles, calculant ainsi le coefficient d'extinction pour la partie de zones partielles.

2. Dispositif de calcul de coefficient selon la revendication 1, comportant en outre :
un moyen de sélection de champ (206) pour sélectionner, en tant que partie de zones partielles, des zones partielles présentant un degré de dispersion d'informations de position supérieur à un degré de dispersion prédéterminé parmi la pluralité de zones partielles, les informations de position représentant des positions des zones partielles.

3. Dispositif de calcul de coefficient selon la revendication 1, comportant en outre :
un moyen de sélection de champ (206) pour sélectionner, dans le cadre de zones partielles, des zones partielles présentant un degré de dispersion des valeurs caractéristiques supérieur à un degré de dispersion prédéterminé parmi la pluralité de zones partielles.

**4.** Dispositif de calcul de coefficient selon l'une quelconque des revendications 1 à 3, dans lequel le moyen de génération d'informations de pertinence (305) est configuré pour

classer les paires coefficient d'extinction/valeur caractéristique pour les zones partielles respectives en une pluralité de groupes selon un procédé de regroupement,
générer la fonction pour chacun des groupes classés, et
générer des informations de position qui indiquent une position moyenne de positions de zones partielles par rapport aux paires coefficient d'extinction/valeur caractéristique appartenant à chaque groupe pour chaque groupe, sur la base d'informations de position indiquant des positions desdites zones partielles, et
le second moyen de calcul de coefficient (304) est configuré pour
sélectionner un groupe, et
calculer le coefficient d'extinction pour l'autre zone partielle sur la base de la fonction générée pour le groupe sélectionné,
le groupe sélectionné étant le plus proche, en matière de position, de l'autre zone partielle parmi la pluralité de groupes.

**5.** Dispositif de calcul de coefficient selon l'une quelconque des revendications 1 à 4, comportant en outre :
un moyen de simulation (107) pour calculer l'indice de zone de feuilles sur la base d'une quantité d'irrigation dans le champ.

**6.** Dispositif de calcul de coefficient selon l'une quelconque des revendications 1 à 4, comportant en outre :
un moyen de calcul d'indice (106) pour calculer un indice de zone de feuilles pour l'autre zone partielle sur la base du coefficient d'extinction calculé par le second moyen de calcul de coefficient (104 ; 204 ; 304) pour l'autre zone partielle et un indice de végétation pour l'autre zone partielle.

**7.** Procédé de calcul de coefficient d'extinction par un dispositif de traitement d'informations comportant :

la réception d'une image (111) prise conformément à une vue angulaire d'un champ (112) ;
le calcul d'un coefficient d'extinction pour une partie de zones partielles, parmi une pluralité de zones partielles (113) de l'image reçue (111) conformément à un traitement de calcul prédéterminé ;
le calcul, en tant que valeur caractéristique, d'un rapport de zone de zone de feuilles dans chacune des zones partielles (113) à l'aide de l'image reçue (111) ;
la génération d'une fonction entre le coefficient d'extinction calculé pour la partie de zones partielles (113) et la valeur caractéristique calculée pour la partie de zones partielles (113), la fonction étant une fonction exponentielle ou une fonction polynomiale ; et
le calcul d'un coefficient d'extinction pour l'autre zone partielle en appliquant à la fonction générée la valeur caractéristique calculée pour une autre zone partielle, l'autre zone partielle étant différente de la partie de zones partielles,
dans lequel l'étape de calcul d'un coefficient d'extinction pour la partie de zones partielles comporte :
l'obtention d'un indice de végétation pour la partie de zones partielles à l'aide de l'image reçue (111), et
l'application du traitement de calcul prédéterminé à l'indice de végétation obtenu et à un indice de zone de feuilles pour la partie de zones partielles, calculant ainsi le coefficient d'extinction pour la partie de zones partielles.

**8.** Programme de calcul de coefficient amenant un ordinateur à exécuter le procédé selon la revendication 7.

# Fig.1

COEFFICIENT CALCULATION
DEVICE — 101

CHARACTERISTIC VALUE
CALCULATION UNIT — 102

FIRST COEFFICIENT
CALCULATION UNIT — 103

SECOND COEFFICIENT
CALCULATION UNIT — 104

RELEVANCE INFORMATION
GENERATION UNIT — 105

INDEX CALCULATION UNIT — 106

SIMULATION UNIT — 107

# Fig.2

```
                    ┌─────────┐
                    │  START  │
                    └────┬────┘
                         │
┌────────────────────────▼─────────────────────────┐      S101
│   CALCULATE CHARACTERISTIC VALUES FOR PARTIAL AREAS │
└────────────────────────┬─────────────────────────┘
                         │
┌────────────────────────▼─────────────────────────┐      S102
│   CALCULATE EXTINCTION COEFFICIENT FOR A PART OF THE │
│                PARTIAL AREAS                       │
└────────────────────────┬─────────────────────────┘
                         │
┌────────────────────────▼─────────────────────────┐      S103
│  GENERATE RELEVANCE INFORMATION REGARDING EXTINCTION │
│       COEFFICIENT AND CHARACTERISTIC VALUE          │
└────────────────────────┬─────────────────────────┘
                         │
┌────────────────────────▼─────────────────────────┐      S104
│  CALCULATE EXTINCTION COEFFICIENT FOR ANOTHER PARTIAL│
│  AREA DIFFERENT FROM THE PART OF THE PARTIAL AREAS   │
└────────────────────────┬─────────────────────────┘
                         │
                    ┌────▼────┐
                    │   END   │
                    └─────────┘
```

# Fig.3

# Fig.4

| PARTIAL AREA ID | CHARACTERISTIC VALUE | EXTINCTION COEFFICIENT |
|---|---|---|
| 1 | 0.9947 | 0.55 |
| 2 | 0.9909 | 0.50 |
| 3 | 0.9834 | 0.47 |
| 4 | 0.9723 | 0.45 |
| 5 | 0.9496 | 0.48 |
| 6 | 0.9186 | 0.40 |
| 7 | 0.9128 | 0.45 |
| 8 | 0.8501 | 0.35 |
| 9 | 0.7977 | 0.37 |
| 10 | 0.7975 | 0.30 |

# Fig.5

$$c = 0.0566e^{2.2002d}$$

Fig.6

COEFFICIENT CALCULATION
DEVICE ⁓ 201

CHARACTERISTIC VALUE
CALCULATION UNIT ⁓ 202

FIRST COEFFICIENT
CALCULATION UNIT ⁓ 203

SECOND COEFFICIENT
CALCULATION UNIT ⁓ 204

RELEVANCE INFORMATION
GENERATION UNIT ⁓ 205

AREA SELECTION UNIT ⁓ 206

INDEX CALCULATION UNIT ⁓ 106

# Fig.7

START

CALCULATE CHARACTERISTIC VALUES FOR PARTIAL AREAS ⟶ S201

SELECT A PART OF THE PARTIAL AREAS ⟶ S202

CALCULATE EXTINCTION COEFFICIENT FOR THE PART OF THE PARTIAL AREAS ⟶ S203

GENERATE RELEVANCE INFORMATION REGARDING EXTINCTION COEFFICIENT AND CHARACTERISTIC VALUE ⟶ S204

CALCULATE EXTINCTION COEFFICIENT FOR ANOTHER PARTIAL AREA DIFFERENT FROM THE PART OF THE PARTIAL AREAS ⟶ S205

END

Fig.8

COEFFICIENT CALCULATION DEVICE — 301

CHARACTERISTIC VALUE CALCULATION UNIT — 302

FIRST COEFFICIENT CALCULATION UNIT — 303

SECOND COEFFICIENT CALCULATION UNIT — 304

RELEVANCE INFORMATION GENERATION UNIT — 305

INDEX CALCULATION UNIT — 106

# Fig.9

```
                          ( START )
```

                                                                    S301
CALCULATE CHARACTERISTIC VALUES FOR PARTIAL AREAS

                                                                    S302
CALCULATE EXTINCTION COEFFICIENT FOR A PART OF THE
PARTIAL AREAS

                                                                    S303
EXECUTE CLASSIFICATION INTO A PLURALITY OF GROUPS

                                                                    S304
GENERATE RELEVANCE INFORMATION REGARDING EXTINCTION
COEFFICIENT AND CHARACTERISTIC VALUE

                                                                    S305
GENERATE POSITION INFORMATION FOR GROUPS

                                                                    S306
SELECT GROUP CLOSE TO ANOTHER PARTIAL AREA DIFFERENT
FROM THE PART OF THE PARTIAL AREAS

                                                                    S307
CALCULATE EXTINCTION COEFFICIENT BASED ON RELEVANCE
INFORMATION FOR SELECTED GROUP

```
                          (  END  )
```

Fig.10

$c = 0.2458e^{0.6814d}$

$c = 0.1249e^{1.2864d}$

Fig.11

COEFFICIENT CALCULATION DEVICE — 401

FIRST COEFFICIENT CALCULATION UNIT — 402

SECOND COEFFICIENT CALCULATION UNIT — 403

RELEVANCE INFORMATION GENERATION UNIT — 404

Fig.12

```
                    ┌─────────┐
                    │  START  │
                    └────┬────┘
                         ↓
┌──────────────────────────────────────────────────────────────┐  ⟋ S401
│   CALCULATE EXTINCTION COEFFICIENT FOR A PART OF PARTIAL       │
│                         AREAS                                  │
└───────────────────────────────┬──────────────────────────────┘
                                 ↓
┌──────────────────────────────────────────────────────────────┐  ⟋ S402
│   GENERATE RELEVANCE INFORMATION REGARDING EXTINCTION          │
│       COEFFICIENT AND CHARACTERISTIC VALUE                     │
└───────────────────────────────┬──────────────────────────────┘
                                 ↓
┌──────────────────────────────────────────────────────────────┐  ⟋ S403
│   CALCULATE EXTINCTION COEFFICIENT FOR ANOTHER PARTIAL         │
│   AREA DIFFERENT FROM THE PART OF PARTIAL AREAS                │
└───────────────────────────────┬──────────────────────────────┘
                                 ↓
                           ┌─────────┐
                           │   END   │
                           └─────────┘
```

Fig.13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010117327 A

**Non-patent literature cited in the description**

- **G.S. CAMPBELL**. Extinction coefficients for radiation in plant canopies calculated using an ellipsoidal inclination angle distribution. *AGRICULTURAL AND FOREST METEOROLOGY.*, 01 April 1986, vol. 36 (4), 317-321